Europäisches Patentamt

European Patent Office

Office européen des brevets

(11) Veröffentlichungsnummer: **0 264 738 B1**

(12) **EUROPÄISCHE PATENTSCHRIFT**

(45) Veröffentlichungstag der Patentschrift: **11.03.92**

(21) Anmeldenummer: **87114762.5**

(22) Anmeldetag: **09.10.87**

(51) Int. Cl.⁵: **A61B 17/22**

(54) **Lithotripsie-Arbeitsplatz.**

(30) Priorität: **22.10.86 DE 3635974**

(43) Veröffentlichungstag der Anmeldung:
**27.04.88 Patentblatt 88/17**

(45) Bekanntmachung des Hinweises auf die
Patenterteilung:
**11.03.92 Patentblatt 92/11**

(84) Benannte Vertragsstaaten:
**DE FR GB NL**

(56) Entgegenhaltungen:
**EP-A- 0 206 331**
**EP-A- 0 238 772**
**DE-U- 8 528 785**
**FR-A- 1 087 356**
**US-A- 4 137 777**

(73) Patentinhaber: **SIEMENS AKTIENGESELL-
SCHAFT
Wittelsbacherplatz 2
W-8000 München 2(DE)**

(72) Erfinder: **Noske, Erich
Ruhsteinweg 12
W-8525 Weiher(DE)**
Erfinder: **Plisek, Franz
Dompfaffstrasse 143
W-8520 Erlangen(DE)**
Erfinder: **Rattner, Manfred
Am Eichengarten 8
W-8520 Buckenhof(DE)**

## Beschreibung

Die Erfindung betrifft einen Lithotripsie-Arbeitsplatz mit einem Patientenlagerungstisch und einem verstellbar gelagerten Stoßwellengenerator, der mit einer Membran am Patienten anlegbar ist.

Aus der DE-U-85 28 785 ist ein Lithotripsie-Arbeitsplatz bekannt, bei dem unter dem Patientenlagerungstisch zwei verstellbare Stoßwellengeneratoren angeordnet sind, von denen jeweils einer der jeweils zu behandelnden Niere entsprechend durch eine Öffnung im Patientenlagerungstisch von unten am Patienten angelegt wird. Eine Zweiebenen-Röntgeneinrichtung dient dabei zur Ortung des jeweils zu behandelnden Steines.

Aus der zwar prioritätsälteren, jedoch nicht vorveröffentlichten EP-A-0 238 772 sowie der ebenfalls prioritätsälteren, jedoch nicht vorveröffentlichten EP-A-0 206 331 ist es bekannt, bei einem Lithotripsie-Arbeitsplatz mit einem Patientenlagerungstisch und einem verstellbar gelagerten Stoßwellengenerator, der mit einer Membran am Patienten anlegbar ist, den Stoßwellengenerator über dem Patientenlagerungstisch verstellbar zu lagern.

Aus der FR-A-1 087 356 ist es bekannt, eine Operationsleuchte und einen Röntgenstrahler in bezug auf einen Operationstisch individuell verstellbar an Deckenstativen anzubringen. Dabei besteht die Möglichkeit nicht nur die Operationsleuchte, sondern auch den Röntgenstrahler oberhalb des Operationstisches zu positionieren.

Der Erfindung liegt die Aufgabe zugrunde, einen Lithotripsie-Arbeitsplatz der eingangs genannten Art gegenüber dem Stand der Technik zu vereinfachen, insbesondere so auszubilden, daß mit Hilfe eines einzigen Stoßwellengenerators wahlweise Steine in verschiedenen Lagen im Patienten, z.B. Steine in der linken und rechten Niere, behandelt werden können.

Diese Aufgabe ist erfindungsgemäß dadurch gelöst, daß der Stoßwellengenerator über dem Patientenlagerungstisch verstellbar gelagert ist und daß der Stoßwellengenerator an einem über dem Patientenlagerungstisch um eine vertikale Achse drehbaren Halter befestigt und derart ausgebildet ist, daß sich seine Längsachse mit der etwa durch die Mitte des Patientenlagerungstisches verlaufenden, vertikalen Achse schneidet. Beim erfindungsgemäßen Lithotripsie-Arbeitsplatz genügt ein einziger Stoßwellengenerator für die Behandlung unterschiedlicher Steine, da er über dem Patientenlagerungstisch in eine jeweils geeignete Lage gebracht werden kann. Ferner ist eine Aussparung im Patientenlagerungstisch nicht erforderlich, weil der Stoßwellengenerator nicht von unten, sondern von oben am Patienten angelegt wird. Außerdem kann der Stoßwellengenerator mit Hilfe des Halters um die vertikale Achse geschwenkt und damit in die jeweils gewünschte Lage gebracht werden.

Eine zweckmäßige Weiterbildung der Erfindung, bei der eine besonders universelle Einstellung des Stoßwellengenerators möglich ist, besteht darin, daß der Stoßwellengenerator in Richtung seiner Längsachse und in vertikaler Richtung verstellbar am Halter gelagert ist. Die Versorgungseinheit für den Stoßwellengenerator kann im Halter untergebracht werden, so daß sich ein besonders kompakter Aufbau des gesamten Arbeitsplatzes ergibt.

Zur Ortung des jeweiligen Steines kann ein Ortungssystem mit zwei Röntgeneinheiten vorhanden sein, deren Zentralstrahlen in einer vertikalen Ebene liegen, in der auch die Längsachse des Patientenlagerungstisches liegt. Das Ortungssystem und der Halter für den Stoßwellengenerator können dabei eine gemeinsame Aufhängung über dem Patientenlagerungstisch besitzen. Es ist aber auch möglich, den Halter und das Ortungssystem individuell über dem Patientenlagerungstisch aufzuhängen. Eine andere Ausgestaltung der Erfindung besteht darin, daß der Halter an einem auf dem Fußboden verfahrbaren Stativ befestigt ist. Er übergreift in diesem Fall den Patientenlagerungstisch. Ferner ist es vorteilhaft, den Stoßwellengenerator so am Halter zu lagern, daß der Winkel zwischen seiner Achse und der vertikalen Achse einstellbar ist. Dadurch ist eine optimale Einstellung des Stoßwellengenerators möglich. Schließlich ist es auch möglich, den Stoßwellengenerator austauschbar am Halter zu befestigen. Zur Ortung können eine oder beide Röntgeneinheiten durch ein Ultraschall-Ortungssystem ergänzt oder ersetzt werden.

Die Erfindung ist nachfolgend anhand von in der Zeichnung dargestellten Ausführungsbeispielen näher erläutert. Es zeigen:

Fig. 1 und 2     zwei Ansichten eines Lithotripsie-Arbeitsplatzes nach der Erfindung, und

Fig. 3     eine der Fig. 1 entsprechende Ansicht einer Variante des Lithotripsie-Arbeitsplatzes gemäß den Fig. 1 und 2.

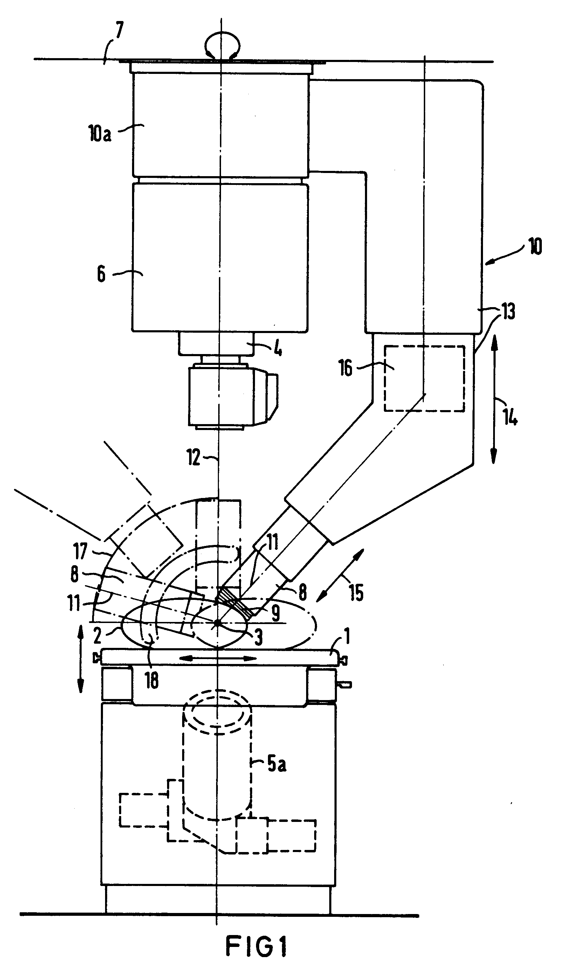

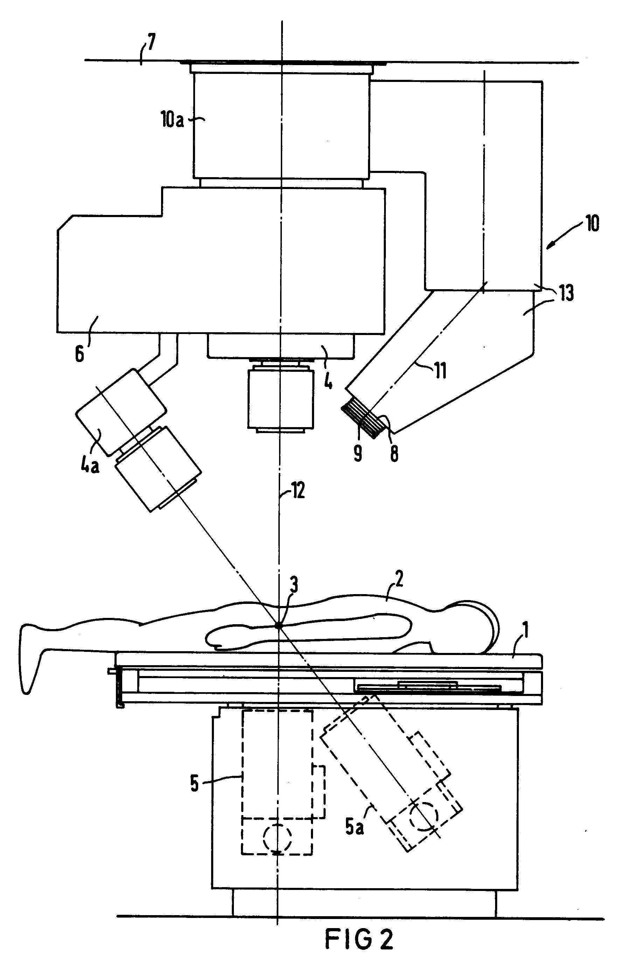

In den Fig. 1 und 2 ist ein Patientenlagerungstisch 1 dargestellt, auf dem ein Patient 2 in Bauchlage liegt, bei dem ein Stein im Isozentrum 3, und zwar ein Nierenstein, zertrümmert werden soll. Zur Ortung des Nierensteines ist eine Zweiebenen-Röntgenuntersuchungseinrichtung mit je einem Röntgenstrahler 4, 4a und einem Röntgenbildverstärker 5, 5a mit nachgeschalteter Fernsehkette vorgesehen, wie dies in dem deutschen Gebrauchsmuster G 85 28 785.7 beschrieben ist. In der Fig. 1 sind der Röntgenstrahler 4 und der Röntgenbildverstärker 5a dargestellt. Der zweite Röntgenstrahler 4a (Fig. 2) liegt in Fig. 1 hinter dem Röntgenstrahler 4 und der zweite Röntgenbildverstärker 5 in Fig. 1 hinter dem Bildverstärker 5a.

Die Röntgenstrahler 4, 4a sind an einem Gehäuse 6 in vertikaler Richtung verstellbar aufgehängt, das an einem Stativ 7 befestigt ist. Zur Zertrümmerung von Steinen im Körper des Patienten 2 ist ein Stoßwellengenerator 8 mit einer am Patienten 2 anlegbaren Membran 9 vorgesehen, der an einem Halter 10 so befestigt ist, daß sich seine Längsachse 11 mit der vertikalen Achse 12 schneidet. Die vertikale Achse 12 geht etwa durch die Mitte des Patientenlagerungstisches 1 und auch durch die Mitte des Röntgenstrahlers 4. Der Halter 10 ist mittels eines Lagers 10a um diese vertikale Achse 12 um einen Winkel von 180° schwenkbar. Der Stoßwellengenerator 8 ist mit dem Halter 10 in Richtung seiner Längsachse 11 verstellbar verbunden (Doppelpfeil 15). Ferner ist der vertikale Teil 13 des Halters 10 teleskopartig in Richtung des Doppelpfeiles 14, also in vertikaler Richtung, ausziehbar, so daß der Stoßwellengenerator 8 auch in vertikaler Richtung verstellbar ist.

Zur Behandlung eines Steines im Körper des Patienten 2 wird der Stoßwellengenerator 8 durch Verschwenkung des Halters 10 um die Achse 12 zunächst auf diejenige Seite des Patienten 2 gebracht, auf der der Stein liegt. Anschließend wird durch Einstellung des Stoßwellengenerators 8 in Richtung der Doppelpfeile 14, 15 der zu behandelnde Stein in den Fokus des Stoßwellengenerators 8 gebracht. Nunmehr kann die Erzeugung von Stoßwellen zur Zertrümmerung des Steines erfolgen.

In der voll dargestellten Stellung des Stoßwellengenerators 8 ist eine Behandlung des auf der linken Seite des Patienten 2 liegenden Nierensteines möglich. Der Patient befindet sich dabei in Bauchlage. Soll ein Stein in der rechten Niere behandelt werden, so wird der Halter 10 um die vertikale Achse 12 um 180° geschwenkt. Mit Hilfe eines einzigen Stoßwellengenerators ist demgemäß ohne Umlagerung des Patienten 2 die Behandlung von Steinen in unterschiedlichen Lagen möglich. Zusätzlich zu einer perkutanen Litholapaxie (PCL) ist die Anwendung einer Stoßwelle möglich. Auch die Behandlung und Zertrümmerung von Gallensteinen ist durchführbar. Hierfür wird der Patient in die Rückenlage gebracht. Der Patientenlagerungstisch 1 ist ein einfacher Tisch, der relativ schmal ausgebildet werden kann. Der Halter 10 kann, wie dargestellt, an einem Stativ aber auch an der Decke des Behandlungsraumes gehaltert sein.

Ist eine Stoßwellenbehandlung nicht vorgesehen, so kann der Stoßwellengenerator 8 in Richtung der Doppelpfeile 14, 15 in eine obere Parkposition verstellt werden (Fig. 2), in der er nicht stört. Die Versorgungseinheit 16 für den Stoßwellengenerator 8 ist im vertikalen Teil 13 des Halters 10 platzsparend untergebracht.

Eine Ergänzung des Ausführungsbeispieles gemäß den Fig. 1 und 2 besteht darin, daß, wie in der Fig. 1 strichpunktiert dargestellt, der Stoßwellengenerator 8 derart verstellbar mit dem Halter 10 verbunden ist, daß der Winkel zwischen seiner Achse 11 und der Achse 12 einstellbar ist. Hierzu kann gemäß Fig. 1 mit dem Halter 10 an dessen Ende eine Führung 17 verbunden sein, die eine Führungsbahn 18 aufweist, deren Mittelpunkt im Isozentrum 3 liegt. Durch Verschwenken des Stoßwellengenerators 8 auf der Führungsbahn 18 ist demgemäß die Einstellung des genannten Winkels und damit eine universelle Einstellung möglich.

In der Fig. 1 ist der untere Teil des Halters 10 auf der linken Seite der Achse 12 strichpunktiert dargestellt, um die Variante mit der Führung 17 und dem verstellbaren Stoßwellengenerator 8 (ebenfalls strichpunktiert gezeichnet) zu zeigen. Auf der linken Seite des Stoßwellengenerators 8 erfolgt natürlich die Behandlung eines Konkrementes, das im Patienten 2 ebenfalls links liegt. Der Patient 2 wird demgemäß dabei aus seiner gezeichneten Stellung nach rechts verschoben.

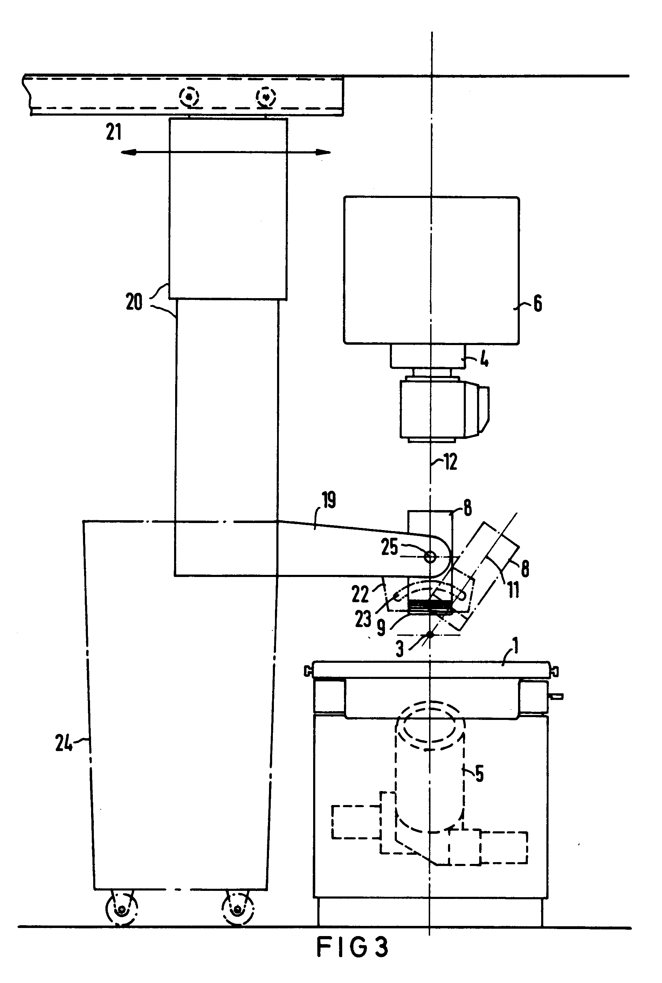

In der Fig. 3 sind Teile, die mit Teilen der Fig. 1 und 2 gleich sind, mit den gleichen Bezugszeichen bezeichnet. Der Stoßwellengenerator 8 ist an einem Arm 19 um eine horizontale Achse 25 schwenkbar befestigt, welcher an einem teleskopartig ausziehbaren Stativ 20 befestigt ist, das in Deckenschienen 21 verfahrbar ist. Die Einstellung des Stoßwellengenerators 8 erfolgt dabei durch Verfahren des Statives 20 und durch Schwenkung des Stoßwellengenerators 8 um seine horizontale Achse 25 in der Weise, daß seine Längsachse 11 immer durch das Isozentrum 3 verläuft.

Eine alternative Einstellmöglichkeit für den Winkel zwischen der Längsachse 11 des Stoßwellengenerators 8 und der Achse 12 besteht darin, daß mit dem Arm 19 eine Führung 22 mit einer Führungsbahn 23 verbunden ist, wobei die Führungsbahn 23 um das Isozentrum 3 gekrümmt ist. Dadurch ist der Stoßwellengenerator 8 hinsichtlich des Winkels zwischen den Achsen 11 und 12 einstellbar, ohne daß eine Verstellung des Statives 20 auf den Schienen 21 erfolgen muß, die bei der Ausführung mit der Achse 25 erforderlich ist.

Die Fig. 3 zeigt, daß eine Abwandlung darin besteht, den Arm 19 mit einem auf dem Fußboden verfahrbaren Stativ 24 zu verbinden.

Allen Ausführungsbeispielen ist gemeinsam, daß ein Ortungssystem mit zwei Röntgeneinheiten 4, 5, 4a, 5a vorhanden ist, deren Zentralstrahlen in einer vertikalen Ebene liegen, in der auch die Längsachse des Patientenlagerungstisches 1 liegt. Bei dem Beispiel gemäß den Fig. 1 und 2 besitzen das Ortungssystem 4, 5, 4a, 5a und der Halter 10 für den Stoßwellengenerator 8 eine gemeinsame Aufhängung über dem Patientenlagerungstisch 2.

Bei dem Ausführungsbeispiel gemäß Fig. 3 sind das Ortungssystem 4, 5, 4a, 5a und der Stoßwellengenerator 8 individuell über dem Patientenlagerungstisch 1 aufgehängt.

Es ist möglich, den Stoßwellengenerator austauschbar zu haltern. Dies kann beispielsweise bei dem Beispiel gemäß der Fig. 3 dadurch erfolgen, daß die Achse 25 lösbar ist.

## Patentansprüche

1. Lithotripsie-Arbeitsplatz mit einem Patientenlagerungstisch (1) und einem verstellbar gelagerten Stoßwellengenerator (8), der mit einer Membran (9) am Patienten (2) anlegbar ist, **dadurch gekennzeichnet,** daß der Stoßwellengenerator (8) über dem Patientenlagerungstisch (1) verstellbar gelagert ist und daß der Stoßwellengenerator (8) an einem über dem Patientenlagerungstisch (1) um eine vertikale Achse (12) drehbaren Halter (10) befestigt und derart ausgerichtet ist, daß sich seine Längsachse (11) mit der etwa durch die Mitte des Patientenlagerungstisches (1) verlaufenden vertikalen Achse (12) schneidet.

2. Lithotripsie-Arbeitsplatz nach Anspruch 1, **dadurch gekennzeichnet,** daß der Stoßwellengenerator (8) in Richtung seiner Längsachse (11) verstellbar am Halter (10) gelagert ist.

3. Lithotripsie-Arbeitsplatz nach Anspruch 1 oder 2, **dadurch gekennzeichnet,** daß der Stoßwellengenerator (8) am Halter (10) in vertikaler Richtung verstellbar ist.

4. Lithotripsie-Arbeitsplatz nach einem der Ansprüche 1 bis 3, **dadurch gekennzeichnet,** daß die Versorgungseinheit (16) für den Stoßwellengenerator (8) im Halter (10) untergebracht ist.

5. Lithotripsie-Arbeitsplatz nach einem der Ansprüche 1 bis 4, **dadurch gekennzeichnet,** daß ein Ortungssystem mit zwei Röntgeneinheiten (4, 5, 4a, 5a) vorhanden ist, deren Zentralstrahlen in einer vertikalen Ebene liegen, in der auch die Längsachse des Patientenlagerungstisches (1) liegt, und daß das Ortungssystem und der Halter (10) eine gemeinsame Aufhängung (10a) über dem Patientenlagerungstisch (1) besitzen.

6. Lithotripsie-Arbeitsplatz nach einem der Ansprüche 1 bis 4, **dadurch gekennzeichnet,** daß ein Ortungssystem mit zwei Röntgeneinheiten (4, 5, 4a, 5a) vorhanden ist, deren Zentralstrahlen in einer vertikalen Ebene liegen, in der auch die Längsachse des Patientenlagerungstisches (1) liegt und daß das Ortungssystem und der Stoßwellengenerator (8) individuell über dem Patientenlagerungstisch (1) aufgehängt sind.

7. Lithotripsie-Arbeitsplatz nach einem der Ansprüche 1 bis 4, **dadurch gekennzeichnet,** daß der Stoßwellengenerator (8) an einem auf dem Fußboden verfahrbaren Stativ (24) befestigt ist.

8. Lithotripsie-Arbeitsplatz nach einem der Ansprüche 1 bis 7, **dadurch gekennzeichnet,** daß der Stoßwellengenerator (8) so gelagert ist, daß der Winkel zwischen seiner Achse (11) und der vertikalen Achse (12) einstellbar ist.

9. Lithotripsie-Arbeitsplatz nach einem der Ansprüche 1 bis 8, **dadurch gekennzeichnet,** daß der Stoßwellengenerator (8) austauschbar gehaltert ist.

10. Lithotripsie-Arbeitsplatz nach einem der Ansprüche 1 bis 4, **dadurch gekennzeichnet,** daß zur Ortung mindestens ein Ultraschall-Ortungssystem über dem Patientenlagerungstisch (1) angeordnet ist.

## Claims

1. Lithotripsy work station having a patient-support table (1) and an adjustably mounted shock wave generator (8) which can be applied to the patient (2) with a membrane (9), characterised in that the shock wave generator (8) is adjustably mounted above the patient-support table (1) and in that the shock wave generator (8) is secured to a holder (10), which is rotatable about a vertical axis (12) above the patient-support table (1), and is aligned such that its longitudinal axis (11) intersects the vertical axis (12) running substantially through the centre of the patient-support table (1).

2. Lithotripsy work station according to claim 1, characterised in that the shock wave generator (8) is mounted on the holder (10) so as to be adjustable in the direction of its longitudinal axis (11).

3. Lithotripsy work station according to claim 1 or 2, characterised in that the shock wave generator (8) can be adjusted on the holder (10) in the vertical direction.

4. Lithotripsy work station according to one of the claims 1 to 3, characterised in that the supply unit (16) for the shock wave generator (8) is accommodated in the holder (10).

5. Lithotripsy work station according to one of the claims 1 to 4, characterised in that a locating system is present with two X-ray units (4, 5, 4a, 5a), the central rays of which lie in a vertical plane in which there also lies the longitudinal axis of the patient-support table (1) and in that the locating system and the holder (10) have a common suspension (10a) above the patient-support table (1).

6. Lithotripsy work station according to one of the claims 1 to 4, characterised in that a locating system is present with two X-ray units (4, 5, 4a, 5a), the central rays of which lie in a vertical plane in which there also lies the longitudinal axis of the patient-support table (1) and in that the locating system and the shock wave generator (8) are suspended individually above the patient-support table (1).

7. Lithotripsy work station according to one of the claims 1 to 4, characterised in that the shock wave generator (8) is secured to a stand (24) which can be moved on the floor.

8. Lithotripsy work station according to one of the claims 1 to 7, characterised in that the shock wave generator (8) is mounted so that the angle between its axis (11) and the vertical axis (12) is adjustable.

9. Lithotripsy work station according to one of the claims 1 to 8, characterised in that the shock wave generator (8) is held in a manner such that it is capable of being replaced.

10. Lithotripsy work station according to one of the claims 1 to 4, characterised in that an ultrasonic locating system is arranged above the patient-support table (1) for the purposes of location.

**Revendications**

1. Poste de travail pour lithotritie comportant un plateau (1) formant couchette pour patient et un générateur d'ondes de choc (8) monté de manière à être déplaçable et pouvant être appliqué au moyen d'une membrane (9) contre le patient (2), caractérisé par le fait que le générateur d'ondes de choc (8) est monté de manière à être déplaçable au-dessus du plateau (1) formant couchette pour patient, est fixé à un support (10) pouvant tourner autour d'un axe vertical (12) au-dessus du plateau (1) formant couchette pour patient et est orienté de telle sorte que son axe longitudinal (11) recoupe l'axe vertical (12) qui passe approximativement au centre du plateau (1) formant couchette pour patient.

2. Poste de travail pour lithotritie suivant la revendication 1, caractérisé par le fait que le générateur d'ondes de choc (8) est monté sur le support (10) de manière à être déplaçable dans la direction de son axe longitudinal (11).

3. Poste de travail pour lithotritie suivant la revendication 2, caractérisé par le fait que le générateur d'ondes de choc (8) est déplaçable verticalement sur le support (10).

4. Poste de travail pour lithotritie suivant l'une des revendications 1 à 3, caractérisé par le fait que l'unité d'alimentation (16) pour le générateur d'ondes de choc (8) est logée dans le support (10).

5. Poste de travail pour lithotritie suivant l'une des revendications 1 à 4, caractérisé par le fait qu'il est prévu un système de repérage comportant deux unités radiologiques (4,5,4a,5a), dont les rayons centraux sont situés dans le plan vertical dans lequel est également situé l'axe longitudinal du plateau (1) formant couchette pour patient, et que le système de repérage et le support (10) possèdent une suspension commune (10a) au-dessus du plateau (1) formant couchette pour patient.

6. Poste de travail pour lithotritie suivant l'une des revendications 1 à 4, caractérisé par le fait qu'il est prévu un système de repérage comportant deux unités radiologiques (4,5,4a,5a), dont les rayons centraux sont situés dans un plan vertical, dans lequel est également situé l'axe longitudinal du plateau (1) formant couchette pour patient et que le système de repérage et le générateur d'ondes de choc (8) sont suspendus individuellement au-dessus du plateau (1) formant couchette pour patient.

7. Poste de travail pour lithotritie suivant l'une des revendications 1 à 4, caractérisé par le fait que le générateur d'ondes de choc (8) est fixé sur un statif (24) déplaçable sur le sol.

8. Poste de travail pour lithotritie suivant l'une des revendications 1 à 7, caractérisé par le fait que le générateur d'ondes de choc (8) est monté de telle sorte que l'angle entre son axe (11) et l'axe vertical (12) est réglable.

**9.** Poste de travail pour lithotritie suivant l'une des revendications 1 à 8, caractérisé par le fait que le générateur d'ondes de choc (8) est monté de manière à être interchangeable.

**10.** Poste de travail pour lithotritie suivant l'une des revendications 1 à 4, caractérisé par le fait que pour le repérage, au moins un système de repérage à ultrasons est disposé au-dessus du plateau (1) formant couchette pour patient.

FIG 1

FIG 2

FIG 3